# FASCICULE DE BREVET EUROPEEN

(11) **EP 1 198 577 B1**
(45) Date de publication et mention de la délivrance du brevet: **18.10.2006**
(21) Numéro de dépôt: 00956593.8
(22) Date de dépôt: 02.08.2000
(51) Int. Cl.: C12N 15/82, C07K 14/415, A01H 5/00

(54) **GENE MUTANT DE LA FAMILLE GRAS, ET PLANTES A DEVELOPPEMENT REDUIT COMPRENANT LEDIT GENE**
MUTIERTES GEN DER GRAS FAMILIE UND PFLANZEN MIT REDUZIERTER ENTWICKLUNG, DIE DIESES GEN ENHALTEN
MUTANT GENE OF THE GRAS FAMILY AND PLANTS WITH REDUCED DEVELOPMENT CONTAINING SAID MUTANT GENE

(30) Priorité: 02.08.1999 FR 9910023
(43) Date de publication de la demande: 24.04.2002
(73) Titulaire: INSTITUT NATIONAL DE LA RECHERCHE AGRONOMIQUE, 75007 Paris (FR)
(72) Inventeur: RENARD, Michel, F-35650 Le Rheu (FR); DELOURME, Régine, F-35590 L'Hermitage (FR); BARRET, Pierre, F-63000 Clermont-Ferrand (FR); BRUNEL, Dominique, F-75012 Paris (FR); FROGER, Nicole, F-78210 Saint-Cyr-l'Ecole (FR); TANGUY, Xavier, F-35650 Moigne le Rheu (FR)
(74) Mandataire: Vialle-Presles, Marie José
(86) Numéro de dépôt international: PCT/FR2000/002216
(87) Numéro de publication internationale: WO 2001/009356

(56) Documents cités:
- WO-A-97/29123
- WO-A-99/09174
- TROUNG ET AL: "Sequence and characterization of two Arabidopsis thaliana cDNAs isolated by functional complementation of a yeast gln3 gdh1 mutant" FEBS LETTERS, vol. 410, 1 juin 1997 (1997-06-01), pages 213-218, XP002088384 ISSN: 0014-5793
- DATABASE TREMBL [en ligne] EMBL Heidelberg, Germany; AC/ID O65367, 1 août 1998 (1998-08-01) MAY M J: "Cloning of the Arabidopsis thaliana RGA-like gene, a putative member of the VHIID domain transcription factor family" XP002135004
- FOISSET N ET AL.: "Molecular tagging of the dwarf BREIZH (Bzh) gene in Brassica napus" THEORETICAL AND APPLIED GENETICS, vol. 91, no. 5, octobre 1995 (1995-10), pages 756-761, XP000901048 cité dans la demande

### LISTE DE SEQUENCES

<110> INRA
   RENARD, Michel
   DELOURME, Régine
   BARRET, Pierre
   BRUNEL, Dominique
   FROGER, Nicole
   TANGUY, Xavier
<120> GENE MUTANT DE LA FAMILLE GRAS, ET PLANTES A DEVELOPPEMENT REDUIT COMPRENANT LEDIT GENE
<130> MJPcb539/95
<140>
   <141>
<150> FR 9910023
   <151> 1999-08-02
<160> 4
<170> PatentIa Ver. 2.1
<210> 1
   <211> 1779
   <212> ADN
   <213> Brassica napus
<220>
   <221> CDS
   <222> (60) . . (1778)
<400> 1
   caacccagaa caaaaccaga ccgatctgag agattaacta tatcttaacc agatcagaa  59
   atg aag agg gat ctt cat cag ttc caa ggt ccc aac cac ggg aca tca   107
   Met Lys Arg Asp Leu His Gln Phe Gln Gly Pro Asn His Gly Thr Ser
    1               5                  10                  15
   atc gcc ggt tct tcc act tct tcc cct gcg gtg ttt ggt aaa gac aag   155
   Ile Ala Gly Ser Ser Thr Ser Ser Pro Ala Val Phe Gly Lys Asp Lys
               20                  25                  30
   atg atg atg gtc aaa gaa gaa gaa gac gac gag ctt cta gga gtc ttg   203
   Met Met Met Val Lys Glu Glu Glu Asp Asp Glu Leu Leu Gly Val Leu
           35                  40                  45
   ggt tac aag gtt agg tct tcg gag atg gct gag gtt gcg ttg aaa ctc   251
   Gly Tyr Lys Val Arg Ser Ser Glu Met Ala Glu Val Ala Leu Lys Leu
       50                  55                  60
   gag cag ctt gag acg atg atg ggt aac gct caa gaa gac ggt tta gct   299
   Glu Gln Leu Glu Thr Met Met Gly Asn Ala Gln Glu Asp Gly Leu Ala
   65                  70                  75                  80
   cac ctc gcg acg gat act gtt cat tac aac ccc gct gag ctt tac tcg   347
   His Leu Ala Thr Asp Thr Val His Tyr Asn Pro Ala Glu Leu Tyr Ser
                   85                  90                  95
   tgg ctt gat aac atg ctc acg gag ctt aac cca ccc gct gca acg acc    395
   Trp Leu Asp Asn Met Leu Thr Glu Leu Asn Pro Pro Ala Ala Thr Thr
               100                 105                 110
   gga tct aac gct ttg aac ccg gag att aat aat aat aat aat aac tcg    443
   Gly Ser Asn Ala Leu Asn Pro Glu Ile Asn Asn Asn Asn Asn Asn Ser
           115                 120                 125
   ttt ttc acc gga ggc gac ctc aaa gcg att cct gga aac gcg gtt tgt    491
   Phe Phe Thr Gly Gly Asp Leu Lys Ala Ile Pro Gly Asn Ala Val Cys
       130                 135                 140
   cgc aga tct aat cag ttc gcg ttt gcg gtt gat tcg tcg agt aat aag    539
   Arg Arg Ser Asn Gln Phe Ala Phe Ala Val Asp Ser Ser Ser Asn Lys
   145                 150                 155                 160
   cgt ttg aaa ccg tcc tcg agc cct gat tcg atg gtt aca tct cca tca    587
   Arg Leu Lys Pro Ser Ser Ser Pro Asp Ser Met Val Thr Ser Pro Ser
                   165                 170                 175
   cct gct gga gtt ata gga acg acg gtt aca acc gtg acc gag tca act    635
   Pro Ala Gly Val Ile Gly Thr Thr Val Thr Thr Val Thr Glu Ser Thr
               180                 185                 190
   cgt cct tta atc ctg gtc gac tcg cag gac aac gga gtg cgt cta gtc    683
   Arg Pro Leu Ile Leu Val Asp Ser Gln Asp Asn Gly Val Arg Leu Val
           195                 200                 205
   cac gcg ctt atg gcc tgc gct gaa gcc gtg cag agc agc aac ttg act    731
   His Ala Leu Met Ala Cys Ala Glu Ala Val Gln Ser Ser Asn Leu Thr
       210                 215                 220
   cta gcg gag gct ctc gtt aag cag att ggt ttc ttg gcc gtc tct caa    779
   Leu Ala Glu Ala Leu Val Lys Gln Ile Gly Phe Leu Ala Val Ser Gln
   225                 230                 235                 240
   gcc gga gcc atg agg aaa gtc gcc acg tac ttc gcc gaa gct ctc gcg    827
   Ala Gly Ala Met Arg Lys Val Ala Thr Tyr Phe Ala Glu Ala Leu Ala
                   245                 250                 255
   cgg agg atc tac cgc ctc tct ccg ccg cag acg cag atc gat cac tct    875
   Arg Arg Ile Tyr Arg Leu Ser Pro Pro Gln Thr Gln Ile Asp His Ser
               260                 265                 270
   tta tcc gat act ctc cag atg cac ttc tac gag act tgc cct tac ctc    923
   Leu Ser Asp Thr Leu Gln Met His Phe Tyr Glu Thr Cys Pro Tyr Leu
           275                 280                 285
   aag ttc gct cac ttc acg gcg aat cag gcg att ctc gag gct ttc gaa    971
   Lys Phe Ala His Phe Thr Ala Asn Gln Ala Ile Leu Glu Ala Phe Glu
       290                 295                 300
   ggg aag aag aga gtc cac gtc atc gat ttc tcg atg aac caa ggg ctt    1019
   Gly Lys Lys Arg Val His Val Ile Asp Phe Ser Met Asn Gln Gly Leu
   305                 310                 315                 320
   cag tgg ccc gcg ctt atg caa gcc ctt gcg ttg agg gaa gga ggt cct    1067
   Gln Trp Pro Ala Leu Met Gln Ala Leu Ala Leu Arg Glu Gly Gly Pro
                   325                 330                 335
   ccg agt ttc agg tta acc gga att ggt cct ccc gcg gcg gat aac tcc    1115
   Pro Ser Phe Arg Leu Thr Gly Ile Gly Pro Pro Ala Ala Asp Asn Ser
               340                 345                 350
   gat cat ctc cat gaa gtt gga tgt aag ttg gct cag ctc gcg gag gcg    1163
   Asp His Leu His Glu Val Gly Cys Lys Leu Ala Gln Leu Ala Glu Ala
           355                 360                 365
   att cac gtc gag ttt gag tat cgt ggc ttt gtt gct aat agc tta gct    1211
   Ile His Val Glu Phe Glu Tyr Arg Gly Phe Val Ala Asn Ser Leu Ala
       370                 375                 380
   gat ctt gat gcc tcg atg ctt gag ctt aga ccg agt gaa acc gaa gct    1259
   Asp Leu Asp Ala Ser Met Leu Glu Leu Arg Pro Ser Glu Thr Glu Ala
   385                 390                 395                 400
   gtg gcg gtt aac tct gtt ttc gag ctc cac aag ctc cta ggc cgt acc    1307
   Val Ala Val Asn Ser Val Phe Glu Leu His Lys Leu Leu Gly Arg Thr
                   405                 410                 415
   ggt ggg ata gag aaa gtc ttc ggc gtt gtg aaa cag att aaa ccg gtg    1355
   Gly Gly Ile Glu Lys Val Phe Gly Val Val Lys Gln Ile Lys Pro Val
               420                 425                 430
   att ttc acg gtt gtt gag caa gaa tcg aat cat aac ggt ccg gtt ttc    1403
   Ile Phe Thr Val Val Glu Gln Glu Ser Asn His Asn Gly Pro Val Phe
           435                 440                 445
   tta gac cgg ttt act gaa tcg ctg cat tat tat tcg acg ttg ttt gat    1451
   Leu Asp Arg Phe Thr Glu Ser Leu His Tyr Tyr Ser Thr Leu Phe Asp
       450                 455                 460
   tcc ttg gaa ggt gct ccg agt agc caa gat aaa gtt atg tcg gaa gtt    1499
   Ser Leu Glu Gly Ala Pro Ser Ser Gln Asp Lys Val Met Ser Glu Val
   465                 470                 475                 480
   tat tta ggg aaa cag att tgc aat ctg gtg gct tgc gaa ggt ccg gac    1547
   Tyr Leu Gly Lys Gln Ile Cys Asn Leu Val Ala Cys Glu Gly Pro Asp
                   485                 490                 495
   cgt gtt gag aga cat gag acg ctg agt caa tgg tcg aac cgg ttc ggt    1595
   Arg Val Glu Arg His Glu Thr Leu Ser Gln Trp Ser Asn Arg Phe Gly
               500                 505                 510
   tcg tcc ggt ttt gcg ccg gcg cat ctc ggg tct aac gcg ttt aag caa    1643
   Ser Ser Gly Phe Ala Pro Ala His Leu Gly Ser Asn Ala Phe Lys Gln
           515                 520                 525
   gcg agt acg ctt ttg gct ttg ttt aat gga ggc gaa ggt tat cgt gtg    1691
   Ala Ser Thr Leu Leu Ala Leu Phe Asn Gly Gly Glu Gly Tyr Arg Val
       530                 535                 540
   gag gag aat aat ggg tgt ttg atg ttg agt tgg cac act cga ccg ctc    1739
   Glu Glu Asn Asn Gly Cys Leu Met Leu Ser Trp His Thr Arg Pro Leu
   545                 550                 555                 560
   ata acc acc tcc gct tgg aag ctc tcg gcg gtg cac tga g              1779
   Ile Thr Thr Ser Ala Trp Lys Leu Ser Ala Val His
                   565                 570
<210> 2
   <211> 572
   <212> PRT
   <213> Brassica napus
<400> 2
   Met Lys Arg Asp Leu His Gln Phe Gln Gly Pro Asn His Gly Thr Ser
    1               5                  10                  15
   Ile Ala Gly Ser Ser Thr Ser Ser Pro Ala Val Phe Gly Lys Asp Lys
               20                  25                  30
   Met Met Met Val Lys Glu Glu Glu Asp Asp Glu Leu Leu Gly Val Leu
           35                  40                  45
   Gly Tyr Lys Val Arg Ser Ser Glu Met Ala Glu Val Ala Leu Lys Leu
       50                  55                  60
   Glu Gln Leu Glu Thr Met Met Gly Asn Ala Gln Glu Asp Gly Leu Ala
   65                  70                  75                  80
   His Leu Ala Thr Asp Thr Val His Tyr Asn Pro Ala Glu Leu Tyr Ser
                   85                  90                  95
   Trp Leu Asp Asn Met Leu Thr Glu Leu Asn Pro Pro Ala Ala Thr Thr
               100                 105                 110
   Gly Ser Asn Ala Leu Asn Pro Glu Ile Asn Asn Asn Asn Asn Asn Ser
           115                 120                 125
   Phe Phe Thr Gly Gly Asp Leu Lys Ala Ile Pro Gly Asn Ala Val Cys
       130                 135                 140
   Arg Arg Ser Asn Gln Phe Ala Phe Ala Val Asp Ser Ser Ser Asn Lys
   145                 150                 155                 160
   Arg Leu Lys Pro Ser Ser Ser Pro Asp Ser Met Val Thr Ser Pro Ser
                   165                 170                 175
   Pro Ala Gly Val Ile Gly Thr Thr Val Thr Thr Val Thr Glu Ser Thr
               180                 185                 190
   Arg Pro Leu Ile Leu Val Asp Ser Gln Asp Asn Gly Val Arg Leu Val
           195                 200                 205
   His Ala Leu Met Ala Cys Ala Glu Ala Val Gln Ser Ser Asn Leu Thr
       210                 215                 220
   Leu Ala Glu Ala Leu Val Lys Gln Ile Gly Phe Leu Ala Val Ser Gln
   225                 230                 235                 240
   Ala Gly Ala Met Arg Lys Val Ala Thr Tyr Phe Ala Glu Ala Leu Ala
                   245                 250                 255
   Arg Arg Ile Tyr Arg Leu Ser Pro Pro Gln Thr Gln Ile Asp His Ser
               260                 265                 270
   Leu Ser Asp Thr Leu Gln Met His Phe Tyr Glu Thr Cys Pro Tyr Leu
           275                 280                 285
   Lys Phe Ala His Phe Thr Ala Asn Gln Ala Ile Leu Glu Ala Phe Glu
       290                 295                 300
   Gly Lys Lys Arg Val His Val Ile Asp Phe Ser Met Asn Gln Gly Leu
   305                 310                 315                 320
   Gln Trp Pro Ala Leu Met Gln Ala Leu Ala Leu Arg Glu Gly Gly Pro
                   325                 330                 335
   Pro Ser Phe Arg Leu Thr Gly Ile Gly Pro Pro Ala Ala Asp Asn Ser
               340                 345                 350
   Asp His Leu His Glu Val Gly Cys Lys Leu Ala Gln Leu Ala Glu Ala
           355                 360                 365
   Ile His Val Glu Phe Glu Tyr Arg Gly Phe Val Ala Asn Ser Leu Ala
       370                 375                 380
   Asp Leu Asp Ala Ser Met Leu Glu Leu Arg Pro Ser Glu Thr Glu Ala
   385                 390                 395                 400
   Val Ala Val Asn Ser Val Phe Glu Leu His Lys Leu Leu Gly Arg Thr
                   405                 410                 415
   Gly Gly Ile Glu Lys Val Phe Gly Val Val Lys Gln Ile Lys Pro Val
               420                 425                 430
   Ile Phe Thr Val Val Glu Gln Glu Ser Asn His Asn Gly Pro Val Phe
           435                 440                 445
   Leu Asp Arg Phe Thr Glu Ser Leu His Tyr Tyr Ser Thr Leu Phe Asp
       450                 455                 460
   Ser Leu Glu Gly Ala Pro Ser Ser Gln Asp Lys Val Met Ser Glu Val
   465                 470                 475                 480
   Tyr Leu Gly Lys Gln Ile Cys Asn Leu Val Ala Cys Glu Gly Pro Asp
                   485                 490                 495
   Arg Val Glu Arg His Glu Thr Leu Ser Gln Trp Ser Asn Arg Phe Gly
               500                 505                 510
   Ser Ser Gly Phe Ala Pro Ala His Leu Gly Ser Asn Ala Phe Lys Gln
           515                 520                 525
   Ala Ser Thr Leu Leu Ala Leu Phe Asn Gly Gly Glu Gly Tyr Arg Val
       530                 535                 540
   Glu Glu Asn Asn Gly Cys Leu Met Leu Ser Trp His Thr Arg Pro Leu
   545                 550                 555                 560
   Ile Thr Thr Ser Ala Trp Lys Leu Ser Ala Val His
                   565                 570
<210> 3
   <211> 1779
   <212> ADN
   <213> Brassica napus
<220>
   <221> CDS
   <222> (60)..(1778)
<400> 3
   caacccagaa caaaaccaga ccgatctgag agattaacta tatcttaacc agatcagaa   59
   atg aag agg gat ctt cat cag ttc caa ggt ccc aac cac ggg aca tca    107
   Met Lys Arg Asp Leu His Gln Phe Gln Gly Pro Asn His Gly Thr Ser
   1               5                   10                  15
   atc gcc ggt tct tcc act tct tcc cct gcg gtg ttt ggt aaa gac aag    155
   Ile Ala Gly Ser Ser Thr Ser Ser Pro Ala Val Phe Gly Lys Asp Lys
               20                  25                  30
   atg atg atg gtc aaa gaa gaa gaa gac gac gag ctt cta gga gtc ttg    203
   Met Met Met Val Lys Glu Glu Glu Asp Asp Glu Leu Leu Gly Val Leu
           35                  40                  45
   ggt tac aag gtt agg tct tcg gag atg gct gag gtt gcg ttg aaa ctc    251
   Gly Tyr Lys Val Arg Ser Ser Glu Met Ala Glu Val Ala Leu Lys Leu
       50                  55                  60
   gag cag ctt gag acg atg atg ggt aac gct caa gaa gac ggt tta gct    299
   Glu Gln Leu Glu Thr Met Met Gly Asn Ala Gln Glu Asp Gly Leu Ala
   65                  70                  75                  80
   cac ctc gcg acg gat act gtt cat tac aac ccc gct gag ctt tac tcg    347
   His Leu Ala Thr Asp Thr Val His Tyr Asn Pro Ala Glu Leu Tyr Ser
                   85                  90                  95
   tgg ctt gat aac atg ctc acg gag ctt aac cca ccc gct gca acg acc    395
   Trp Leu Asp Asn Met Leu Thr Glu Leu Asn Pro Pro Ala Ala Thr Thr
               100                 105                 110
   gga tct aac gct ttg aac ccg gag att aat aat aat aat aat aac tcg    443
   Gly Ser Asn Ala Leu Asn Pro Glu Ile Asn Asn Asn Asn Asn Asn Ser
           115                 120                 125
   ttt ttc acc gga ggc gac ctc aaa gcg att cct gga aac gcg gtt tgt    491
   Phe Phe Thr Gly Gly Asp Leu Lys Ala Ile Pro Gly Asn Ala Val Cys
       130                 135                 140
   cgc aga tct aat cag ttc gcg ttt gcg gtt gat tcg tcg agt aat aag    539
   Arg Arg Ser Asn Gln Phe Ala Phe Ala Val Asp Ser Ser Ser Asn Lys
   145                 150                 155                 160
   cgt ttg aaa ccg tcc tcg agc cct gat tcg atg gtt aca tct cca tca    587
   Arg Leu Lys Pro Ser Ser Ser Pro Asp Ser Met Val Thr Ser Pro Ser
                   165                 170                 175
   cct gct gga gtt ata gga acg acg gtt aca acc gtg acc gag tca act    635
   Pro Ala Gly Val Ile Gly Thr Thr Val Thr Thr Val Thr Glu Ser Thr
               180                 185                 190
   cgt cct tta atc ctg gtc gac tcg cag gac aac gga gtg cgt cta gtc    683
   Arg Pro Leu Ile Leu Val Asp Ser Gln Asp Asn Gly Val Arg Leu Val
           195                 200                 205
   cac gcg ctt atg gcc tgc gct gaa gcc gtg cag agc agc aac ttg act    731
   His Ala Leu Met Ala Cys Ala Glu Ala Val Gln Ser Ser Asn Leu Thr
       210                 215                 220
   cta gcg gag gct ctc gtt aag cag att ggt ttc ttg gcc gtc tct caa    779
   Leu Ala Glu Ala Leu Val Lys Gln Ile Gly Phe Leu Ala Val Ser Gln
   225                 230                 235                 240
   gcc gga gcc atg agg aaa gtc gcc acg tac ttc gcc gaa gct ctc gcg    827
   Ala Gly Ala Met Arg Lys Val Ala Thr Tyr Phe Ala Glu Ala Leu Ala
                   245                 250                 255
   cgg agg atc tac cgc ctc tct ccg ccg cag acg cag atc gat cac tct    875
   Arg Arg Ile Tyr Arg Leu Ser Pro Pro Gln Thr Gln Ile Asp His Ser
               260                 265                 270
   tta tcc gat act ctc cag atg cac ttc tac gag act tgc cct tac ctc    923
   Leu Ser Asp Thr Leu Gln Met His Phe Tyr Glu Thr Cys Pro Tyr Leu
           275                 280                 285
   aag ttc gct cac ttc acg gcg aat cag gcg att ctc gag gct ttc gaa    971
   Lys Phe Ala His Phe Thr Ala Asn Gln Ala Ile Leu Glu Ala Phe Glu
       290                 295                 300
   ggg aag aag aga gtc cac gtc atc gat ttc tcg atg aac caa ggg ctt   1019
   Gly Lys Lys Arg Val His Val Ile Asp Phe Ser Met Asn Gln Gly Leu
   305                 310                 315                 320
   cag tgg ccc gcg ctt atg caa gcc ctt gcg ttg agg gaa gga ggt cct   1067
   Gln Trp Pro Ala Leu Met Gln Ala Leu Ala Leu Arg Glu Gly Gly Pro
                   325                 330                 335
   ccg agt ttc agg tta acc gga att ggt cct ccc gcg gcg gat aac tcc   1115
   Pro Ser Phe Arg Leu Thr Gly Ile Gly Pro Pro Ala Ala Asp Asn Ser
               340                 345                 350
   gat cat ctc cat gaa gtt gga tgt aag ttg gct cag ctc gcg gag gcg   1163
   Asp His Leu His Glu Val Gly Cys Lys Leu Ala Gln Leu Ala Glu Ala
           355                 360                 365
   att cac gtc gag ttt gag tat cgt ggc ttt gtt gct aat agc tta gct   1211
   Ile His Val Glu Phe Glu Tyr Arg Gly Phe Val Ala Asn Ser Leu Ala
       370                 375                 380
   gat ctt gat gcc tcg atg ctt gag ctt aga ccg agt gaa acc gaa gct   1259
   Asp Leu Asp Ala Ser Met Leu Glu Leu Arg Pro Ser Glu Thr Glu Ala
   385                 390                 395                 400
   gtg gcg gtt aac tct gtt ttc gag ctc cac aag ctc cta ggc cgt acc   1307
   Val Ala Val Asn Ser Val Phe Glu Leu His Lys Leu Leu Gly Arg Thr
                   405                 410                 415
   ggt ggg ata gag aaa gtc ttc ggc gtt gtg aaa cag att aaa ccg gtg   1355
   Gly Gly Ile Glu Lys Val Phe Gly Val Val Lys Gln Ile Lys Pro Val
               420                 425                 430
   att ttc acg gtt gtt gag caa gaa tcg aat cat aac ggt ccg gtt ttc   1403
   Ile Phe Thr Val Val Glu Gln Glu Ser Asn His Asn Gly Pro Val Phe
           435                 440                 445
   tta gac cgg ttt act gaa tcg ctg cat tat tat tcg acg ttg ttt gat    1451
   Leu Asp Arg Phe Thr Glu Ser Leu His Tyr Tyr Ser Thr Leu Phe Asp
       450                 455                 460
   tcc ttg gaa ggt gct ccg agt agc caa gat aaa gtt atg tcg gaa gtt    1499
   Ser Leu Glu Gly Ala Pro Ser Ser Gln Asp Lys Val Met Ser Glu Val
   465                 470                 475                 480
   tat tta ggg aaa cag att tgc aat ctg gtg gct tgc gaa ggt ccg gac    1547
   Tyr Leu Gly Lys Gln Ile Cys Asn Leu Val Ala Cys Glu Gly Pro Asp
                   485                 490                 495
   cgt gtt gag aga cat gag acg ctg agt caa tgg tcg aac cgg ttc ggt    1595
   Arg Val Glu Arg His Glu Thr Leu Ser Gln Trp Ser Asn Arg Phe Gly
               500                 505                 510
   tcg tcc ggt ttt gcg ccg gcg cat ctc ggg tct aac gcg ttt aag caa    1643
   Ser Ser Gly Phe Ala Pro Ala His Leu Gly Ser Asn Ala Phe Lys Gln
           515                 520                 525
   gcg agt acg ctt ttg gct ttg ttt aat gga ggc gaa ggt tat cgt gtg    1691
   Ala Ser Thr Leu Leu Ala Leu Phe Asn Gly Gly Glu Gly Tyr Arg Val
       530                 535                 540
   gag aag aat aat ggg tgt ttg atg ttg agt tgg cac act cga ccg ctc    1739
   Glu Lys Asn Asn Gly Cys Leu Met Leu Ser Trp His Thr Arg Pro Leu
   545                 550                 555                 560
   ata acc acc tcc gct tgg aag ctc tcg gcg gtg cac tga g              1779
   Ile Thr Thr Ser Ala Trp Lys Leu Ser Ala Val His
                   565                 570
<210> 4
   <211> 572
   <212> PRT
   <213> Brassica napus
<400> 4
   Met Lys Arg Asp Leu His Gln Phe Gln Gly Pro Asn His Gly Thr Ser
    1               5                  10                  15
   Ile Ala Gly Ser Ser Thr Ser Ser Pro Ala Val Phe Gly Lys Asp Lys
               20                  25                  30
   Met Met Met Val Lys Glu Glu Glu Asp Asp Glu Leu Leu Gly Val Leu
           35                  40                  45
   Gly Tyr Lys Val Arg Ser Ser Glu Met Ala Glu Val Ala Leu Lys Leu
       50                  55                  60
   Glu Gln Leu Glu Thr Met Met Gly Asn Ala Gln Glu Asp Gly Leu Ala
   65                  70                  75                  80
   His Leu Ala Thr Asp Thr Val His Tyr Asn Pro Ala Glu Leu Tyr Ser
                   85                  90                  95
   Trp Leu Asp Asn Met Leu Thr Glu Leu Asn Pro Pro Ala Ala Thr Thr
               100                 105                 110
   Gly Ser Asn Ala Leu Asn Pro Glu Ile Asn Asn Asn Asn Asn Asn Ser
           115                 120                 125
   Phe Phe Thr Gly Gly Asp Leu Lys Ala Ile Pro Gly Asn Ala Val Cys
       130                 135                 140
   Arg Arg Ser Asn Gln Phe Ala Phe Ala Val Asp Ser Ser Ser Asn Lys
   145                 150                 155                 160
   Arg Leu Lys Pro Ser Ser Ser Pro Asp Ser Met Val Thr Ser Pro Ser
                   165                 170                 175
   Pro Ala Gly Val Ile Gly Thr Thr Val Thr Thr Val Thr Glu Ser Thr
               180                 185                 190
   Arg Pro Leu Ile Leu Val Asp Ser Gln Asp Asn Gly Val Arg Leu Val
           195                 200                 205
   His Ala Leu Met Ala Cys Ala Glu Ala Val Gln Ser Ser Asn Leu Thr
       210                 215                 220
   Leu Ala Glu Ala Leu Val Lys Gln Ile Gly Phe Leu Ala Val Ser Gln
   225                 230                 235                 240
   Ala Gly Ala Met Arg Lys Val Ala Thr Tyr Phe Ala Glu Ala Leu Ala
                   245                 250                 255
   Arg Arg Ile Tyr Arg Leu Ser Pro Pro Gln Thr Gln Ile Asp His Ser
               260                 265                 270
   Leu Ser Asp Thr Leu Gln Met His Phe Tyr Glu Thr Cys Pro Tyr Leu
           275                 280                 285
   Lys Phe Ala His Phe Thr Ala Asn Gln Ala Ile Leu Glu Ala Phe Glu
       290                 295                 300
   Gly Lys Lys Arg Val His Val Ile Asp Phe Ser Met Asn Gln Gly Leu
   305                 310                 315                 320
   Gln Trp Pro Ala Leu Met Gln Ala Leu Ala Leu Arg Glu Gly Gly Pro
                   325                 330                 335
   Pro Ser Phe Arg Leu Thr Gly Ile Gly Pro Pro Ala Ala Asp Asn Ser
               340                 345                 350
   Asp His Leu His Glu Val Gly Cys Lys Leu Ala Gln Leu Ala Glu Ala
           355                 360                 365
   Ile His Val Glu Phe Glu Tyr Arg Gly Phe Val Ala Asn Ser Leu Ala
       370                 375                 380
   Asp Leu Asp Ala Ser Met Leu Glu Leu Arg Pro Ser Glu Thr Glu Ala
   385                 390                 395                 400
   Val Ala Val Asn Ser Val Phe Glu Leu His Lys Leu Leu Gly Arg Thr
                   405                 410                 415
   Gly Gly Ile Glu Lys Val Phe Gly Val Val Lys Gln Ile Lys Pro Val
               420                 425                 430
   Ile Phe Thr Val Val Glu Gln Glu Ser Asn His Asn Gly Pro Val Phe
           435                 440                 445
   Leu Asp Arg Phe Thr Glu Ser Leu His Tyr Tyr Ser Thr Leu Phe Asp
       450                 455                 460
   Ser Leu Glu Gly Ala Pro Ser Ser Gln Asp Lys Val Met Ser Glu Val
   465                 470                 475                 480
   Tyr Leu Gly Lys Gln Ile Cys Asn Leu Val Ala Cys Glu Gly Pro Asp
                   485                 490                 495
   Arg Val Glu Arg His Glu Thr Leu Ser Gln Trp Ser Asn Arg Phe Gly
               500                 505                 510
   Ser Ser Gly Phe Ala Pro Ala His Leu Gly Ser Asn Ala Phe Lys Gln
           515                 520                 525
   Ala Ser Thr Leu Leu Ala Leu Phe Asn Gly Gly Glu Gly Tyr Arg Val
       530                 535                 540
   Glu Lys Asn Asn Gly Cys Leu Met Leu Ser Trp His Thr Arg Pro Leu
   545                 550                 555                 560
   Ile Thr Thr Ser Ala Trp Lys Leu Ser Ala Val His
                   565                 570

## Description

L'invention est relative à l'obtention de végétaux à développement réduit, et notamment de crucifères.

L'emploi de plantes naines dans le cadre des productions agricoles présente de nombreux avantages ; par exemple, chez les céréales, l'utilisation de plantes mutantes à paille courte a permis d'obtenir des cultures tolérant des quantités importantes d'engrais azotés, moins affectées par les conditions climatiques, et notamment plus résistantes à la verse que les plantes de taille normale. En outre, la petite taille des plantes facilite l'entretien des cultures, notamment l'application des traitements phytosanitaires, ainsi que leur récolte.

Des mutants nains de végétaux autres que les céréales ont également été décrits dans la littérature. On mentionnera en particulier ci-après des mutants présentant des caractéristiques similaires à celles induites par une déficience en gibbérellines, et insensibles à l'apport de gibbérellines exogènes. De tels mutants ont notamment été décrits chez *Arabidopsis* [KOORNNEEF et al., Physiol. Plant., 65, 33-39, (1985)]. Ces mutants, dénommés *gai* (pour Gibberellic Acid Insensitive) ont une taille réduite et ne répondent pas aux applications exogènes de gibbérellines. La mutation *gai* est une mutation du type « gain de fonction », semi-dominante. Les mutants hétérozygotes *GAI*/*gai* ont un phénotype intermédiaire entre celui des mutants nains *gai*/*gai* et des plantes sauvages *GAI*/*Gai.*

Des mutants présentant les mêmes caractéristiques que les mutants *gai* d'*Arabidopsis* ont été décrits par ZANEWICH et al. [J. Plant Growth Regul., 10, 121-127, (1991)], chez *Brassica napus* (mutation *dwf1*) et *Brassica rapa* (mutations dénommées *dwf1* et *dwf2*).

L'équipe des Inventeurs a obtenu un mutant nain de *B. rapa* [FOISSET et al., Theor. Appl. Genet., 91, 756-761, (1995)]. La mutation, dénommée bzh présente des caractéristiques de « semi-dominance » et d'insensibilité aux gibbérellines similaires à celles de la mutation *gai.*

Une lignée de colza dénommée ISN1770, homozygote pour l'allèle mutant bzh, a fait l'objet d'un Certificat d'Obtention Végétale, déposé le 18 mai 1998, auprès du CPOV (11 rue Jean Nicaud, 75007 PARIS) sous la référence 10751. Un hybride de colza, dénommé « LUTIN » (B017), et comprenant dans son génome l'allèle mutant bzh sous forme hétérozygote a été proposé à l'inscription au Catalogue Français des Obtentions Végétales le 31 juillet 1999, sous la référence 072426.

Le gène *GAI d'Arabidopsis* a récemment été cloné et séquencé [PENG et al., Genes and development, 11, 3194-3205, (1997) ; Demande PCT WO 97/29123 au nom de JOHN INNES CENTRE INNOVATIONS LTD]. Ce gène code pour une protéine (GAI) de 532 aa. L'alléle *gai,* responsable du nanisme, contient une délétion de 51 paires de bases en phase avec le cadre de lecture, qui conduit à l'absence de 17 aa situés près de l'extrémité N-terminale de la protéine GAI. La protéine GAI est impliquée dans la perception et la réponse aux gibbérellines, et agirait chez les plantes sauvages, comme un régulateur négatif de l'élongation cellulaire en absence de gibbérellines.

La comparaison de la séquence de GAI avec celle des produits de traduction d'autres gènes connus a permis de la rattacher à la famille dénommée GRAS [PYSH et al., The Plant Journal, 18(1), 11-119, (1999)] ou VHIID [SCHUMACHER et al., P.N.A.S., 96, 1, 290-295, (1999)].

Cette famille englobe, outre *GAI,* les gènes *RGA* (SILVESTRONE et al., Genetics, 146, 1087-1099, (1998)], et *SCARECROW* [DI LAURENZIO et al., Cell, 86, 423-433, (1996)] *d'Arabidopsis,* ainsi que le gène *LS* (Lateral suppressor) de la Tomate [SHUMACHER et al., P.N.A.S., 96, 1, 290-295, (1999)]. A l'heure actuelle, une vingtaine de gènes rattachés à la famille GRAS ont été identifiés chez *Arabidopsis.*

Les protéines constituant la famille GRAS présentent une partie N-terminale très variable et une partie C-terminale très conservée avec cinq motifs reconnaissables, notamment le motif VHIID.

Les fonctions biologiques de la plupart de ces protéines ne sont pas encore précisément connues mais leur rôle comme facteurs de transcription est fortement supposé. Les travaux effectués sur les 4 gènes les mieux étudiés à l'heure actuelle (*SCR, GAI, RGA* et *LS*) montrent que ces gènes codent des facteurs de transcription impliqués dans le contrôle de la perception et de la réponse aux gibbérellines, et indiquent l'importance probable de cette famille sur le contrôle de la morphogènèse et du développement des plantes supérieures.

Les Inventeurs ont maintenant caractérisé et séquencé le gène *BZH* de *B. napus,* et son allèle mutant bzh, associé au phénotype nain précédemment observé par FOISSET et al.(1995, publication précitée).

La séquence du gène BZH sauvage est représentée dans la liste de séquences en annexe sous le numéro SEQ ID NO: 1, et la séquence de son produit de traduction est représentée sous le numéro SEQ ID NO: 2. La séquence de l'allèle mutant bzh est représentée dans la liste de séquences en annexe sous le numéro SEQ ID NO: 3, et la séquence de son produit de traduction est représentée sous le numéro SEQ ID NO: 4.

La portion codante du gène BZH est de 1716 pbs et la protéine correspondante est de 572 acides aminés.

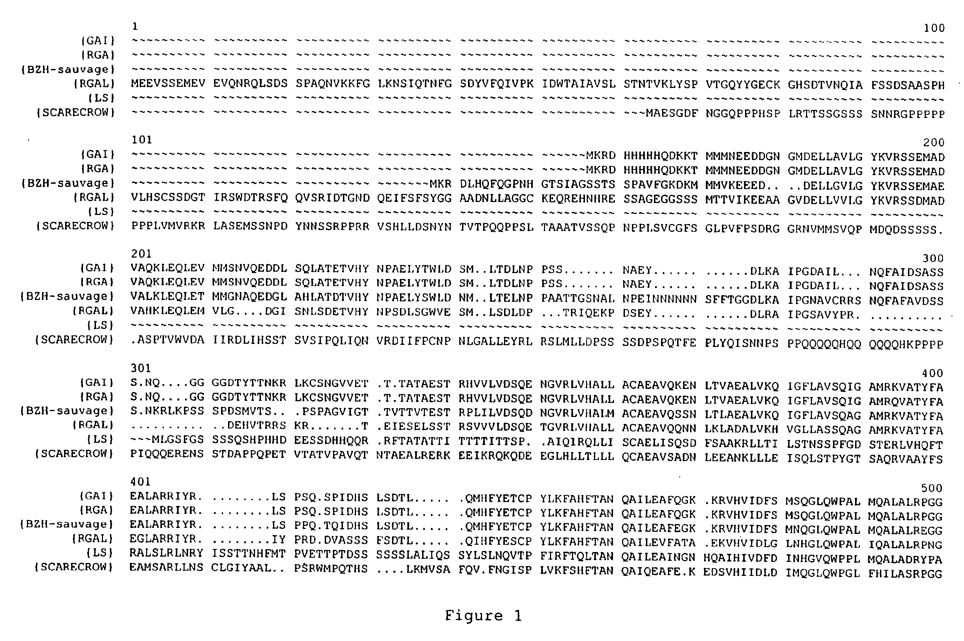

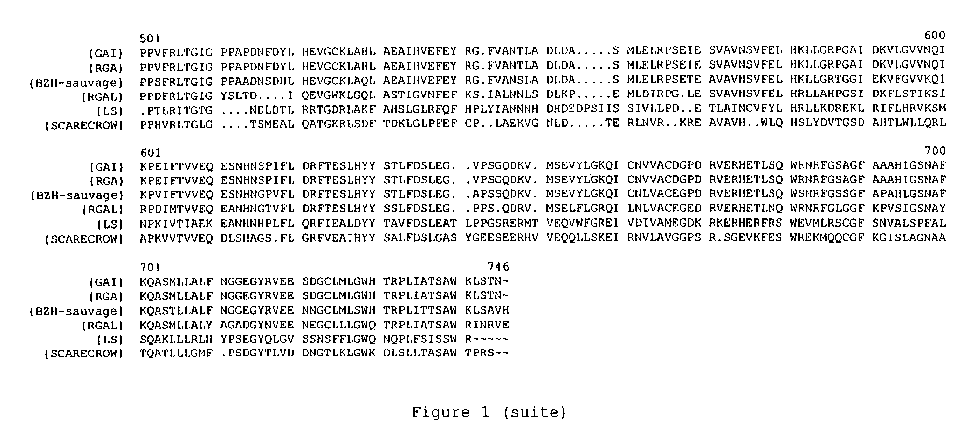

L'analyse des séquences du gène BZH et de son produit de traduction permet de le rattacher à la famille GRAS, et notamment au sous-groupe comprenant GAI, RGA et RGA-like. L'alignement des séquences polypeptidiques déduites des gènes BZH avec d'autres gènes de la famille GRAS, à savoir les gènes *GAI, RGA, RGA-LIKE, SCARECROW* et *LS,* est représenté sur la Figure 1.

L'analyse des séquences de l'allèle mutant bzh et de son produit de traduction montre que la mutation *bzh* est une subtitution G → A en position 1695 de la séquence codante. Elle conduit à un changement d'acide aminé acide glutamique → Lysine en position 546 de la séquence polypeptidique.

De manière surprenante, la mutation bzh est totalement différente de la mutation *gai d'Arabidopsis.* En particulier, alors que la mutation *gai d'Arabidopsis* affecte une région située dans la portion N-terminale de la protéine GAI, la mutation *bzh* affecte une région située dans la portion C-terminale de la protéine BZH.

La présente invention a pour objet une protéine dont l'expression dans une plante entraîne chez ladite plante une réduction de taille insensible aux gibbérellines, caractérisée en ce qu'elle est définie par la séquence SEQ ID NO: 4.

L'invention englobe aussi les séquences d'acide nucléique codant pour une protéine conforme à l'invention, par exemple la séquence de l'allèle mutant bzh qui est représentée dans la liste de séquences en annexe sous le numéro SEQ ID NO:3.

L'invention a également pour objet des procédés d'obtention de plantes à développement réduit comprenant une ou plusieurs copies d'une séquence d'acide nucléique conforme à l'invention, à savoir :
- un procédé d'obtention de plantes mutantes, à partir de plantes sauvages de la famille des Brassicacées comprenant une séquence d'acide nucléique codant pour une protéine définie par la séquence SEQ ID NO: 2, par les techniques classiques de mutagenèse, par exemple en traitant des semences par un agent mutagène physique ou chimique, en sélectionnant parmi les plantes issues des semences traitées, les plantes présentant un nanisme insensible aux gibbérellines, et en sélectionnant parmi celles-ci, par des techniques classiques de détection d'hybridation des acides nucléiques, celles qui comprennent une séquence d'acide nucléique codant pour une protéine conforme à l'invention ;
- un procédé d'obtention de plantes transgéniques, obtenues par transgenèse d'une plante hôte, en particulier une crucifère et avantageusement une brassicacée, avec une séquence d'acide nucléique conforme à l'invention ;

La présente invention a aussi pour objet les plantes à développement réduit exprimant une protéine conforme à l'invention. Il s'agit des plantes mutantes ou des plantes transgéniques susceptibles d'être obtenues par un procédé conforme à l'invention, ainsi que de leurs descendants, pouvant être obtenus par reproduction sexuée ou multiplication végétative.

Avantageusement, des plantes conformes à l'invention sont des brassicacées, choisies parmi le colza et la navette.

Les plantes exprimant une séquence d'acide nucléique conforme à l'invention présentent, par rapport aux plantes sauvages, une réduction de taille plus ou moins importante selon le niveau d'expression dans ladite plante de la séquence d'acide nucléique conforme à l'invention. Ce niveau d'expression dépend en particulier du nombre de copies de la séquence. Par exemple, dans le cas du colza, les plants hétérozygotes *BZH*/*bzh* ont une taille intermédiaire entre celle des plants nains homozygotes *bzh*/*bzh* et celle des plants sauvages *BZH*/*BZH.*

Les plantes conformes à l'invention présentent, notamment dans le cas du colza, les avantages suivants :
- possibilité de semis très précoces, permettant l'assimilation des nitrates, sans risque d'élongation de la tige avant l'hiver ;
- meilleure résistance au froid ;
- meilleur suivi de la culture, du fait d'une taille plus courte facilitant les traitements phytosanitaires ;
- très bonne résistance à la verse ;
- facilité de la récolte.

La présente invention sera mieux comprise à l'aide du complément de description qui va suivre, qui se réfère à des exemples non limitatifs décrivant la caractérisation du gène *BZH* de colza, et d'une séquence conforme à l'invention dérivée dudit gène.

### EXEMPLE 1 : CARACTERISATION ET SEQUENÇAGE DU GENE BZH SAUVAGE ET DU GENE MUTANT bzh:

Le gène BZH a été isolé sur un fragment d'ADN de 2352 paires de bases obtenu à partir de la lignée de colza « STELLAR ». Ce fragment contient une séquence codante de 1716 pbs, et la séquence polypeptidique déduite est de 572 acides aminés. La séquence codante et la séquence polypeptidique déduite sont respectivement représentées sur la liste de séquence en annexe sous les numéros SEQ ID NO: 1 et 2.

Pour comparer la séquence du gène sauvage et de l'allèle mutant *bzh,* 5 lignées ont été étudiées: PRIMOR sauvage (PS), PRIMOR nain (PN), DARMOR sauvage (DS), DARMOR nain (DN) et STELLAR sauvage (STE).

Les fragments d'ADN correspondant au locus BZH ont été amplifiés sur ces lignées, à l'aide d'amorces dérivées de la séquence du SEQ ID NO: 1.

La comparaison des séquences des produits d'amplification obtenus a permis d'établir que la seule différence commune à PRIMOR nain et DARMOR nain par rapport aux génotypes sauvages est une substitution G → A en position 1695 de la séquence codante. Cette substitution conduit à un changement d'acide aminé Glu→Lys en position 546 de la séquence peptidique.

La séquence codante portée par le fragment d'acide nucléique amplifié à partir de la lignée primur nain, et la séquence peptidique correspondante sont respectivement représentées dans la liste de séquences en annexe sous les numéros SEQ ID NO: 3 et 4.

### EXEMPLE 2 : DETECTION DE L'ALLELE MUTANT bzh CHEZ DES PLANTES NAINES.

49 lignées issues du croisement : DARMOR nain X YUDAL, ainsi que les couples de lignées isogéniques [sauvage]/[bzh] suivants : ISL1770/ISN1770, DOUBLOL/DOUBLOL-Bzh, GASPARD/GASPARD-Bzh, TAPIDOR/TAPIDOR-Bzh, ont été analysées par amplification PCR d'une région de 400 pb environ de la séquence codante, correspondant à la portion C-terminale de la protéine, et électrophorèse sur gel de polyacrylamide des produits d'amplification.

Les lignées de phénotype « nain » présentaient sur le gel une bande caractéristique de la présence de la substitution G → A.

## Revendications

1. Protéine dont l'expression dans une plante entraîne chez ladite plante une réduction de taille insensible aux gibbérellines, **caractérisée en ce qu'**elle est définie par la séquence SEQ ID NO: 4.

2. Polynucléotide isolé codant pour une protéine selon la revendication 1.

3. Procédé d'obtention de plantes à développement réduit, à partir de plantes de la famille des Brassicacées comprenant une séquence d'acide nucléique codant pour une protéine définie par la séquence SEQ ID NO: 2, par mutagénèse et sélection de plantes mutantes qui présentent un nanisme insensible aux gibbérellines, **caractérisé en ce qu'**il comprend en outre la sélection, parmi lesdites plantes mutantes, de celles qui comprennent une séquence d'acide nucléique codant pour une protéine selon la revendication 1.

4. Procédé d'obtention d'une plante à développement réduit, **caractérisé en ce qu'**il comprend la transgenèse d'une plante hôte avec un polynucléotide selon la revendication 2.

5. Procédé selon la revendication 4, **caractérisé en ce que** ladite plante est une crucifère.

6. Procédé selon la revendication 4, **caractérisé en ce que** ladite plante est une Brassicacée.

7. Procédé selon une quelconque des revendications 3 ou 6, **caractérisé en ce que** ladite Brassicacée est choisie parmi le colza et la navette.

8. Plante à développement réduit, exprimant une protéine selon la revendication 1, et susceptible d'être obtenue par un procédé selon une quelconque des revendications 3 à 7.

9. Plante à développement réduit, exprimant une protéine selon la revendication 1, descendant d'une plante selon la revendication 8.

## Claims

1. Protein whose expression in a plant brings about in that plant a reduction in size insensitive to gibberellins, **characterised in that** it is defined by the sequence SEQ ID NO: 4.

2. Isolated polynucleotide encoding a protein according to claim 1.

3. Process for obtaining plants having reduced development, from plants of the Brassicaceae family comprising a nucleic acid sequence encoding a protein defined by the sequence SEQ ID NO: 2, by mutagenesis and selection of mutant plants which have a dwarfism insensitive to gibberellins, **characterised in that** it also comprises the selection, from the mutant plants, of those which comprise a nucleic acid sequence encoding a protein according to claim 1.

4. Process for obtaining a plant having reduced development, **characterised in that** it comprises the transgenesis of a host plant with a polynucleotide according to claim 2.

5. Process according to claim 4, **characterised in that** the plant is a crucifer.

6. Process according to claim 4, **characterised in that** the plant is a Brassicaceae.

7. Process according to either claim 3 or claim 6, **characterised in that** the Brassicaceae is selected from colza and rape.

8. Plant having reduced development, expressing a protein according to claim 1, and capable of being obtained by a process according to any one of claims 3 to 7.

9. Plant having reduced development, expressing a protein according to claim 1, descending from a plant according to claim 8.

## Patentansprüche

1. Protein, dessen Expression in einer Pflanze eine Verminderung ihrer Größe bedingt, die gegen Gibberelline unempfindlich ist, **dadurch gekennzeichnet, dass** es durch die Sequenz SEQ ID Nr.: 4 definiert wird.

2. Isoliertes Polynucleotid, das für ein Protein nach Anspruch 1 codiert.

3. Verfahren zum Erhalt von Pflanzen mit verminderter Entwicklung, ausgehend von Pflanzen der Familie der *Brassicaceae,* die eine Nucleinsäuresequenz umfassen, die für ein bestimmtes Protein codiert das durch die Sequenz SEQ ID Nr.: 2 definiert ist, mit Mutagenese und Selektion der mutieren Pflanzen, die einen Zwergwuchs aufweisen, der gegenüber Gibberellinen unempfindlich ist, **dadurch gekennzeichnet, dass** es unter anderem eine Selektion unter den genannten Mutantenpflanzen, die eine Nucleinsäuresequenz umfassen, die für das Protein nach Anspruch 1 codiert, umfasst.

4. Verfahren zum Erhalt einer Pflanze mit verminderter Entwicklung, **dadurch gekennzeichnet, dass** es die Transgenese einer Wirtspflanze mit einem Polynucleodid nach Anspruch 2 umfasst.

5. Verfahren nach Anspruch 4, **dadurch gekennzeichnet, dass** die genannte Pflanze eine Crucifere ist.

6. Verfahren nach Anspruch 4, **dadurch gekennzeichnet, dass** die genannte Pflanze eine Brassicacee ist.

7. Verfahren nach einem der Ansprüche 3 oder 6, **dadurch gekennzeichnet, dass** die genannte Brassicacee ausgewählt ist aus Raps und Rübsen.

8. Pflanze mit verminderter Entwicklung, die ein Protein nach Anspruch 1 exprimiert und die durch ein Verfahren nach einem der Ansprüche 3 bis 7 erhältlich ist.

9. Pflanze mit verminderter Entwicklung, die ein Protein nach Anspruch 1 exprimiert und von einer Pflanze nach Anspruch 8 abstammt.
